(19) Europäisches Patentamt

European Patent Office

Office européen des brevets

(11) Veröffentlichungsnummer: **0 206 291 B1**

(12) **EUROPÄISCHE PATENTSCHRIFT**

(45) Veröffentlichungstag der Patentschrift: **12.06.91**

(21) Anmeldenummer: **86108472.1**

(22) Anmeldetag: **20.06.86**

(51) Int. Cl.⁵: **A61K 9/12,** A61K 47/00, A61K 31/19

(54) **Aufsprühbare pharmazeutische Zubereitung für die topische Anwendung.**

(30) Priorität: **24.06.85 DE 3522550**

(43) Veröffentlichungstag der Anmeldung:
**30.12.86 Patentblatt 86/52**

(45) Bekanntmachung des Hinweises auf die
Patenterteilung:
**12.06.91 Patentblatt 91/24**

(84) Benannte Vertragsstaaten:
**AT BE CH DE FR GB IT LI LU NL SE**

(56) Entgegenhaltungen:
**EP-A- 0 055 397**
**EP-A- 0 091 964**
**EP-A- 0 175 671**
**DE-A- 1 800 580**
**DE-A- 3 305 689**

(73) Patentinhaber: **Klinge Pharma GmbH**
**Berg-am-Laim-Strasse 129**
**W-8000 München 80(DE)**

(72) Erfinder: **Stanislaus, Friedrich**
**Halserspitzstrasse 12**
**W-8000 München 80(DE)**
Erfinder: **Hofer, Josef Maximilian**
**Wasserburgerstrasse 24a**
**W-8011 Kirchseeon(DE)**
Erfinder: **Knoch, Axel**
**Neumarkter Strasse 86d**
**W-8000 München 80(DE)**

(74) Vertreter: **Wuesthoff, Franz, Dr.-Ing. et al**
**Wuesthoff & Wuesthoff Patent- und Rechts-**
**anwälte Schweigerstrasse 2**
**W-8000 München 90(DE)**

## Beschreibung

Eine Therapie mit topisch applizierbaren pharmazeutischen Zubereitungen ist vor allem dann von Vorteil, wenn Applikationsort und Krankheitsprozeß nahe beieinander liegen. So werden zur perkutanen Behandlung von rheumatischen oder traumatischen Affektionen entsprechende Wirkstoffe häufig in Form von Pflastern, Salben, Cremes, Gelen, Emulsionen oder Suspensionen u.ä. eingesetzt.

Diese topischen Anwendungsformen besitzen jedoch eine Reihe von Mängeln, deren Beseitigung einen Fortschritt in der pharmazeutischen Entwicklung bedeutet. So müssen zur Herstellung dieser Präparate meist eine Vielzahl von Hilfsstoffen eingesetzt werden, die eine unnötige Belastung des Organismus darstellen. Trotz der zahlreichen Hilfsstoffe ist die Stabilität dieser Präparate bei höheren Temperaturen meist ungenügend. Dies führt zu einem "Brechen" der Zubereitungen, so daß eine inhomogene Mischung der Präparatebestandteile vorliegt, die eine therapeutische Anwendung nicht mehr ermöglicht. Dies gilt insbesondere für Zubereitungen in Form von Emulsionen oder sonstigen Dispersionen. Auch müssen bei solchen bisher üblichen topischen Anwendungsformen wie z.B. Emulsionen, Suspensionen usw. spezielle Transport- und Verteilungsvorgänge des Wirkstoffes zwischen den verschiedenen Phasen ablaufen, bevor sie durch die Haut in den Körper ein dringen können und wirksam werden. Daher hat man auch bereits die Wirkstoffe in gelöster Form aufgetragen, wobei es sich als vorteilhaft erwiesen hat, wenn die Wirkstoffe in einem Lösungsmittelgemisch aus einem flüchtigen physiologisch verträglichen Lösungsmittel und einem nichtflüchtigen, physiologisch verträglichen Lösgungsmittel gelöst vorliegen. Nach Verdunsten des flüchtigen Lösungsmittels bleibt der Wirkstoff in dem nichtflüchtigen Lösungsmittel auf der Haut in angereicherter, jedoch weiterhin gelöster Form zurück und kann hierdurch besser resorbiert werden.

Aus der EP-A 91 964 ist bereits bekannt, daß sich für diese Gemische eine Kombination aus niedrigem Alkohol als flüchtiger Komponente, gesättigte aliphatische Kohlenwasserstoffe mit 5 - 20 C-Atomen, einem Ester aus einem niedrigen Alkohol und einer Fettsäure mit 13 - 24 C-Atomen oder einer Ätherverbindung mit 8 - 16 C-Atomen als nichtflüchtige Komponente der Lösungsmittelmischung eignen. In der deutschen Patentanmeldung DE-A 33 05 689 ist bei einer topisch anwendbaren Zubereitung aus Isosorbiddinitrat als Wirkstoff die Verwendung eines Gemisches aus einem lipophilem und einem hydrophilem Lösungsmittel in einem Gewichtsverhältnis von 0,8 bis 0,2 offenbart, wobei das lipophile Lösungsmittel Diisopropyladipat, Isopropylmyristat oder Isosorbiddimethyläther sein kann, während als hydrophile Lösungsmittel ein Polyäthylenglykol oder ein Polyhydroxyäthylen-Polyhydroxypropylen-Kondensat geeignet ist. Offenbart ist auch die Verwendung von Äthanol als hydrophiles Lösungsmittel.

Es wurde nun überraschenderweise festgestellt, daß bei einem Lösungsmittelgemisch aus flüchtigem und nichtflüchtigem Lösungsmittel die Verwendung von Propylenglykol oder einem Polyolfettsäureester bzw. deren Mischung als nichtflüchtige Lösungsmittelkomponente sich besondere Vorteile bei der Resorption der Wirkstoffe durch die Haut ergeben. Der Vergleich mit vorbekannten Lösungsmittelgemischen zeigt eine deutlich erhöhte Penetrationsrate der Wirkstoffe, wenn diese nichtflüchtige Lösungsmittelkomponenten in der Zubereitung vorhanden sind.Erfindungsgemäß enthalten daher die aufsprühbaren Zubereitungen als nichtflüchtiges Lösungsmittel Propylenglykol und/oder Polyolfettsäureester, wobei das Verhältnis von flüchtigen und den neuen nichtflüchtigen Lösungsmitteln in der aufsprühbaren Zubereitung im Gewichtsverhältnis von 1:1 bis 20:1 liegen soll. Das jeweils günstigste Verhältnis kann bei dem jeweiligen Wirkstoff leicht durch einige Versuche festgestellt werden, ebenso wie die günstigste Konzentration des Wirkstoffes in der Zubereitung, um dann nach dem Verdunsten des flüchtigen Lösungsmittels eine hochkonzentrierte Wirkstofflösung auf der Haut zu haben, was die Penetration und Resorption fördert.

Durch Zusatz eines in den Lösungsmitteln löslichen Filmbildners wird die Haftung auf der Haut erhöht. Insbesondere Polyacrylate sind hierbei als Filmbildner geeignet. Durch die Wahl der Art und der Menge des Filmbildners kann auch die Resorption verzögert und eine Depotwirkung erreicht werden. Durch Auftragen der Zubereitung auf eine kleinere oder grössere Hautfläche kann man auch in besonders einfacher Weise das Ausmaß der perkutanen Resorption des jeweiligen Wirkstoffs steuern.

Die Erfindung eignet sich besonders bei Sprayzubereitungen von antiphlogistisch wirksamen Phenylalkansäuren wie z.B. DICLOFENAC, FENOPROFEN, FLURBIPROFEN, IBUPROFEN, NAPROXIN oder KETOPROFEN sowie deren Salze. Diese Wirkstoffe werden durch die Haut ausreichend bzw. gut resorbiert und können in der Nähe des erkrankten Gebiets des Körpers aufgetragen, dann schnell zur Wirkung gelangen.

An flüchtigen Lösungsmittelkomponenten in dem erfindungsgemäßen Gemisch mit Propylenglykol und/oder Polyolfettsäureester werden bevorzugt Ethanol oder Isopropanol verwendet. Als nicht-essentielle Bestandteile der erfindungsgemäßen Zubereitung können noch eingesetzt werden üblich Treibmittel für derartige Zubereitungen und Penetrationsförderer, z.B. Dimethylsulfoxid sowie Stabilisatoren, z.B. Buthylhydroxyanisol.

Durch Verwendung von besonderen Dosiereinrichtungen, wie z.B. Pumpzerstäuber, lassen sich die erfindungsgemäßen Zubereitungen mit hoher Dosiergenauigkeit applizieren. Von Vorteil ist die berührungsfreie Auftragung, was von Patienten mit oberflächlich lokalisierten Schmerzen besonders angenehmn empfunden wird. Nach Applikation auf das erkrankte Gebiet wird durch das rasche Abdunsten der flüchtigen Lösungsmittelkomponente ein schmerzlindernder Kühleffekt erzielt. Dabei reichert sich der Wirkstoff in der nichtflüchtigen Lösungsmittelkomponentean, so daß er in erhöhter Konzentration zur Resorption zur Verfügung steht.

Die erfindungsgemäßen pharmazeutischen Zubereitungen können in Verfahren zur äußeren Behandlung von Schmerzzuständen, Entzündungen und/oder rheumatischen Erkrankungen bei Warmblütern eingesetzt werden. Besonders geeignet sind sie zur Behandlung von Sport- und Unfallverletzungen, wie Prellungen, Verstauchungen und Zerrungen; ebenso zur unterstützenden Behandlung bei Muskelrheumatismus, degenerativen schmerzhaften Gelenkerkrankungen (Arthrosen), entzündlichen rheumatischen Erkrankungen der Gelenke und der Wirbelsäule, Schwellung bzw. Entzündung der gelenknahen Weichteile (z.B. Schleimbeutel, Sehnen, Sehnenscheiden, Bänder und Gelenkkapseln), Schultersteife, Kreuzschmerzen und Hexenschuß.

Die nachfolgenden Beispiele dienen zur weiteren Erläuterung der Erfindung; sie sollen diese jedoch in ihrem Umfang nicht einschränken.

Beispiel 1
Sprayzubereitung enthaltend DICLOFENAC-NATRIUM

1 kg DICLOFENAC-NATRIUM wird in 5 kg Propylenglykol gelöst, anschließend mit 44 kg Isopropanol versetzt und nach Klarfiltration in 1.000 Schraubbehälter mit Pumpzerstäuberkopf abgefüllt.

Beispiel 2
Sprayzubereitung enthaltend DICLOFENAC

1 kg DICLOFENAC wird unter Erwärmen in 19 kg Polyolfettsäureester, wie es als CETIOL HE im Handel erhältlich ist, gelöst, anschließend mit 80 kg Isopropanol versetzt und nach Klarfiltration in 2.000 Schraubbehälter mit Pumpzerstäuberkopf abgefüllt.

Beispiel 3
Sprayzubereitung enthaltend IBUPROFEN

2,5 kg IBUPROFEN werden in 10 kg Propylenglykol gelöst, dann mit 37,5 kg Isopropanol versetzt und nach Klarfiltration in 1.000 Schraubbehälter mit Pumpzerstäuberkopf abgefüllt.

Beispiel 4
Sprayzubereitung enthaltend NAPROXEN-NATRIUM

1,0 kg NAPROXEN-NATRIUM wird unter Erwärmen in 20 kg des Polyolfettsäureesters, wie er in Beispiel 2 verwendet wurde, aufgelöst, dann mit 79 kg Isopropanol versetzt und nach Klarfiltration unter Treibmittelzusatz in 1.000 Sprühdosen abgefüllt.

Als "flüchtiges Lösungsmittel" sollen hier solche Lösungsmittel gemeint sein, deren Dampfdruck bei der Hauttemperatur von 32° C über 0,046 Atm (35 mm Hg) (35 x 133,3 Pa) liegt, während als "nichtflüchtiges Lösungsmittel" ein Lösungsmittel zu verstehen ist, dessen Dampfdruck bei 32° C unter 0,013 Atm (10 mm Hg) (10 x 133,3 Pa) liegt. Besonders geeignet sind solche, deren Dampfdruck bei 32° C weniger als 0,0066 Atm (5 mm Hg) (5 x 133,3 Pa), insbesondere 0,0013 - 0,0026 Atm (1 - 2 mm Hg) (1 x 133,3 Pa - 2 x 133,3 Pa) beträgt.

**Ansprüche**

1. Aufsprühbare Zubereitung für die topische Anwendung von pharmazeutischen Wirkstoffen mit einem Gehalt an einer Phenylalkansäure bzw. deren Salze als Wirkstoff, einem Lösungsmittelgemisch aus

   a) einem oder mehreren flüchtigen, physiologisch verträglichen Lösungsmitteln und

   b) einem oder mehreren nichtflüchtigen, physiologisch verträglichen Lösungsmitteln, sowie üblichen Zusätzen, dadurch **gekennzeichnet**, daß als nichtflüchtiges Lösungsmittel Propylenglykol oder ein Polyolfettsäureester oder deren Mischung vorhanden ist, wobei das Gewichtsverhältnis von flüchtigen zu nichtflüchtigen Lösungsmitteln 1:1 bis 20:1 beträgt.

2. Zubereitung nach Anspruch 1, dadurch **gekennzeichnet**, daß sie als Wirkstoff DICLOFENAC, FENOPROFEN, FLURBIPROFEN, IBUPROFEN, NAPROXEN oder KETOPROFEN in einer Konzentration von 0,1% bis 10% enthält.

3. Zubereitung nach Anspruch 1 oder 2, dadurch **gekennzeichnet**,

daß sie eine filmbildende Substanz, insbesondere Polyacrylat enthält.

## Claims

1. A sprayable preparation for topical application of pharmaceutical active agents containing a phenylalcanoic acid or the salts thereof as active agent, a solvent mixture of
   a) one or a plurality of volatile, physiologically compatible solvent(s) and
   b) one or a plurality of non-volatile, physiologically compatible solvent(s),
   as well as conventional additives,
   **characterized** in that popylene glycol or a polyol fatty acid ester or a mixture thereof is present as non-volatile solvent, the weight ratio of volatile to non-volatile solvents being 1:1 to 20:1.

2. The preparation according to claim 1, **characterized** in that it contains DICLOFENAC, FENOPROFEN, FLURBIPROFEN, IBUPROFEN, NAPROXEN or KETOPROFEN as active agent, the concentration being 0.1% to 10%.

3. The preparation according to claim 2, **characterized** in that it contains a film forming substance, especially polyacrylate.

## Revendications

1. Préparation vaporisable, pour l'application topique de substances actives pharmaceutiques, contenant un acide phénylalcanoïque ou ses sels comme substance active, un mélange de solvants constitué
   a) d'un ou de plusieurs solvants volatils physiologiquement compatibles et
   b) d'un ou de plusieurs solvants non volatils physiologiquement compatibles
   ainsi que des additifs usuels,
   caractérisée en ce qu'elle contient, comme solvant non volatil, du propylèneglycol ou un ester d'acides gras et de polyols ou leur mélange, le rapport pondéral du solvant volatil/solvant non volatil allant de 1:1 à 20:1.

2. Préparation selon la revendication 1, caractérisée en ce qu'elle contient comme substance active, le Diclofénac, le Fénoprofène, le Flurbiprofène, l'Ibuprofène, le Naproxène ou le Kétoprofène en une concentration allant de 0,1 % à 10 %.

3. Préparation selon la revendication 1 ou 2, caractérisée en ce qu'elle contient une substance filmogène, en particulier un polyacrylate.